# EUROPEAN PATENT SPECIFICATION

(11) **EP 4 064 737 B1**
(45) Date of publication and mention of the grant of the patent: **20.12.2023**
(21) Application number: 21163865.5
(22) Date of filing: 22.03.2021
(51) Int. Cl.: H04W 4/029, G06F 21/62, G16H 50/80, G08B 21/22

(54) **METHOD FOR ENABLING CONTACT TRACING AND CONTACT ALERTING AMONG A PLURALITY OF PERSONS BY MEANS OF DETECTING OR MEASURING THE DISTANCE BETWEEN TWO PERSONS, SYSTEM, TAG DEVICE, WEARABLE OR PORTABLE DEVICES, PROGRAM AND COMPUTER-READABLE MEDIUM**
VERFAHREN ZUR ERMÖGLICHUNG DER KONTAKTVERFOLGUNG UND KONTAKTWARNUNG ZWISCHEN MEHREREN PERSONEN MITTELS ERFASSUNG ODER MESSUNG DES ABSTANDS ZWISCHEN ZWEI PERSONEN, SYSTEM, KENNZEICHNUNGSVORRICHTUNG, AM KÖRPER TRAGBARE ODER TRAGBARE VORRICHTUNGEN, PROGRAMM UND COMPUTERLESBARES MEDIUM
PROCÉDÉ D'ACTIVATION DE TRAÇAGE DE CONTACT ET D'ALERTE DE CONTACT PARMI UNE PLURALITÉ DE PERSONNES AU MOYEN DE LA DÉTECTION OU DE LA MESURE DE LA DISTANCE ENTRE DEUX PERSONNES, SYSTÈME, DISPOSITIF D'ÉTIQUETTE, DISPOSITIFS PORTATIFS OU PORTABLES, PROGRAMME ET SUPPORT LISIBLE PAR ORDINATEUR

(43) Date of publication of application: 28.09.2022
(73) Proprietor: Deutsche Telekom AG, 53113 Bonn (DE)
(72) Inventor: ROLLIN, Hannes, 19055 Schwerin (DE); QUECK, Oliver, 72813 Sankt Johann - Würtingen (DE)
(74) Representative: Schwöbel, Thilo K.

(56) References cited:
- DE-U1-202020 103 175
- WILLIAM J BUCHANAN ET AL: "Review and Critical Analysis of Privacy-preserving Infection Tracking and Contact Tracing", ARXIV.ORG, CORNELL UNIVERSITY LIBRARY, 201 OLIN LIBRARY CORNELL UNIVERSITY ITHACA, NY 14853, 10 September 2020 (2020-09-10), XP081760207,
- "Comparison of existing pandemic contact tracing systems", ETSI DRAFT; ETSI GR E4P-002 V1.1.1- PUBLISHED VERSION-V1.2.0 INTERNAL, EUROPEAN TELECOMMUNICATIONS STANDARDS INSTITUTE (ETSI), 650, ROUTE DES LUCIOLES ; F-06921 SOPHIA-ANTIPOLIS ; FRANCE , vol. ISG - E4P, no. V1.1.1 17 December 2020 (2020-12-17), pages 1-113, XP014396414, Retrieved from the Internet: URL:ftp://docbox.etsi.org/ISG/E4P/Open/e4p -002v111publishedversion_v120internalversi on.pdf [retrieved on 2020-12-17]

## Description

### BACKGROUND

The present invention relates to a method for enabling contact tracing and contact alerting among a plurality of persons by means of detecting or measuring the distance between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices.

Furthermore, the present invention relates to a system for enabling contact tracing and contact alerting among a plurality of persons by means of detecting or measuring the distance between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices.

Additionally, the present invention relates to a tag device and to wearable or portable devices for enabling contact tracing among a plurality of persons by means of detecting relevant encounters between two persons, wherein each of the plurality of persons are wearing or carrying a wearable or portable device.

Additionally, the present invention relates to a program and to a computer-readable medium for enabling contact tracing and contact alerting among a plurality of persons by means of detecting or measuring the distance between two persons according to the inventive method.

It is conventionally known to realize contact detection and/or contact tracing and/or contact alerting among a plurality of persons by means of detecting or measuring the distance between two persons using wearable or portable devices, especially socalled ultra-wideband wearable or portable devices, or ultra-wideband tags. Such wearable or portable devices are typically able to alert their users in case of close contacts and/or to store log data related to the contact situation of the respective or considered wearable or portable device with other wearable or portable devices being or having been in proximity during a considered time interval such as, e.g., a day or a work shift, thereby providing the possibility to either help to avoid the spread of infectious diseases or at least to be able to detect encounters between at least two persons and later on to trace such contacts or encounters.

However, the use of static thresholds (of the respective distance between wearable or portable devices and/or of the respective time of a close encounter), e.g. for initiating (acoustic) alerts and/or for considering an encounter to be risky is not necessarily appropriate as there are so many different situations where need for warning and infection risk are either higher or lower than supposed by static (or average) values. Documents "Review and Critical Analysis of Privacy-preserving Infection Tracking and Contact Tracing"; DE202020103175U and "Comparison of existing pandemic contact tracing systems" disclose contact tracing as well.

### SUMMARY

An object of the present invention is to provide a technically simple, effective, and cost-effective solution for enabling contact tracing and contact alerting among a plurality of persons by means of detecting or measuring the distance between two persons. A further object of the present invention is to provide a corresponding system, a tag device and wearable or portable devices, wearable or portable devices, and a corresponding program and computer-readable medium. The scopoe of protection sought for is defined by the appendend claims.

The object of the present invention is achieved by a method for enabling contact tracing and contact alerting among a plurality of persons by means of detecting or measuring the distance between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices,
wherein, in a contact situation of a specific wearable or portable device with a specific further wearable or portable device, the specific wearable or portable device is able, by means of the specific wearable or portable device's wireless communication interface and with respect to the specific further wearable or portable device, to repeatedly determine the distance, at different points in time, towards the specific further wearable or portable device and to receive the specific further wearable or portable device's tag identifier,
wherein, in order to detect alert situations and/or in order to detect relevant encounters or to assess the relevance of encounters of the specific wearable or portable device with the specific further wearable or portable device, the method comprises the following steps:
   -- in a first step, at least one proximity event involving the specific further wearable or portable device, and detected by the specific wearable or portable device, is determined by means of detecting the specific further wearable or portable device to be positioned at most at, or below, a specific distance, from the specific wearable or portable device, and this for at least a specific time interval, wherein the proximity event is considered to be an encounter detection event if the specific distance corresponds to a risk distance threshold and the specific time interval corresponds to a risk time interval and/or wherein the proximity event is considered to be an alert event if the specific distance corresponds to an alert distance threshold and the specific time interval corresponds to an alert time interval,
   -- in a second step, prior to, during or after the first step, the specific wearable or portable device additionally, detects or receives
      -- a group size information, the group size information being indicative of the relevant group size and/or of the relevant density of persons, and/or
      -- an environmental information, the environmental information being indicative of a static and/or dynamic environmental parameter or property,
wherein the risk distance threshold and the risk time interval and/or the alert distance threshold and the alert time interval to be applied are dependent on the group size information and/or on the environmental information.

It is thereby advantageously possible according to the present invention to not only effectively detect relevant encounters of two wearable or portable devices and/or detect situations necessitating alerts of the respective users, but also to do that in a manner adapted to the respective situation or type of situation of the encounter.

According to the present invention, it is, hence, advantageously possible to be able to take additional parameters and/or measurements into consideration regarding triggering an alert and/or for assessing the (infection) risk of a person wearing or carrying the wearable or portable device. Hence, it is proposed according to the present invention that the applicable threshold values (i.e. the risk distance threshold together with the risk time interval and/or the alert distance threshold together with the alert time interval) are modified in a manner dependent on the group size information and/or the environmental information.

Thereby, it is advantageously possible to apply threshold values that are more appropriate regarding different specific situations than just constant and average model parameters; hence, according to the present invention, it is advantageously possible to more realistically assess warning and alerting levels as well as risk assessment regarding encounters of individual users, taking into account different situations where the warning level and/or the infection risk is or are either higher or lower than supposed for average situations.

In conventionally known contact or encounter detection and/or alert systems, a static warning or alerting distance and a static warning or alerting duration are applied which might or typically do correspond to values of model parameters that might be appropriate for certain standard situations but would be inappropriate with regard to many other situations.

When using contact detecting and/or tracing tags or wearable or portable devices, these tags or wearable or portable devices allow to combine high-precision contact tracing - especially if ultra-wideband, UWB, wearables or wearable devices or tags are used - especially with a decentralized risk calculation on a computing device, often or typically the users' smartphones. Typically, each wearable or portable device or tag has a unique ID, or tag identifier which is broadcast and saved by other wearable or portable devices or tags in the vicinity. The sum or combination of these measurements gives rise to logs (or log data), which are stored on the wearable or portable device or tag and are typically transported to a computing device, again often a user's smart phone or other personal digital assistant, or part of the wearable or portable device, where the log data must be processed to detect risk encounters.

The wearable or portable devices might either not be personalized per se (and in this case they can be used and reused by anyone (typically among one group (or different groups) of persons concerned, such as, e.g. but not necessarily, the staff of an organization or a differently defined group of persons)), but during one session, only a single user is typically using one wearable or portable device or tag. According to another embodiment of the present invention, the wearable or portable device is able to provide the functionality of both a tag (or wearable device, i.e. primarily directed to contact detection) and of a computing device, i.e. for analyzing the log data.

According to the present invention, normally a certain number of persons is considered such as, especially, the staff of an enterprise or of an organization, or the visitors of an event. It is aimed at enabling contact detection and contact tracing for these persons such that contact tracing is able to be performed individually for each person, and typically performed by each person individually using their personal computing device, especially a smart phone or other personal portable device. However, the wearable or portable device does not necessarily need to be a dedicated contact tracking or detecting device but might also be embodied as, e.g., a mobile phone of the person wearing or carrying it. Likewise, the number of persons is not necessarily strictly limited a priori to, e.g., the staff of an enterprise or of an organization.

During operation, e.g. during a work shift or during a work day or during an event such as a sports event or concert, each person is typically wearing or carrying a wearable or portable device or tag (typically one, but, in principle, it is not excluded to wear more than one tag) of the plurality of wearable or portable devices. To each wearable or portable device is assigned or each wearable or portable device is associated to a tag identifier, i.e. an identifying information such as a serial number or the like. At a specific point in time, each wearable or portable device or each tag is associated to exactly one tag identifier; however, in principle according to the present invention, it is not excluded that one and the same wearable or portable device or tag is able to be associated or assigned (successively) to more than one tag identifier, especially in order to still enhance the level of data protection or data privacy, especially by avoiding to create specific pattern, e.g. regarding the usage of certain wearable or portable devices or tags by certain persons or the like. However, in case that more than one tag identifiers are able to be associated or assigned to one and the same wearable or portable device, a mechanism is needed to securely identify or determine such tag identifier without doubt each time the wearable or portable device is used. In the following, the invention is mainly described under the assumption that a tag identifier is fixedly, i.e. unchangeably, associated or assigned to a wearable or portable device.

According to the present invention, the wearable or portable devices, while in operation, more or less continuously, i.e. repeatedly, broadcast the assigned or associated (at this point in time unique) tag identifier (using the wireless communication interfaces of the respective wearable or portable devices), i.e. these broadcasts are typically performed, by the wearable or portable devices and under normal conditions, by applying a certain rate of broadcasts (i.e. a number of broadcasts per time unit, such as, typically once per second (i.e. 1 Hz) or the like; usually such broadcast rates might range from about 0,1 Hz (i.e. once every 10 seconds) to about 10 Hz (ten broadcasts per second)); a broadcast typically corresponding to a radio frequency signal being emitted by the respective wearable or portable device applying a certain standardized or usual emission power such that another wearable or portable device, located in proximity, is able to receive such radio frequency signal and derive or determine, from some signal parameter (such as, typically, some signal strength parameter) and/or from some signal content (such as, e.g., time stamp information and/or emission power information) an indication regarding the distance between both wearable or portable devices. Of course, a corresponding broadcast and radio frequency signal reception procedure is symmetric and both are occurring simultaneously. This also means that, in case that more than two wearable or portable devices are present or located at a specific location or within a considered area (of a size or extension smaller than the radio frequency coverage area of each of the wearable or portable devices), there might be many broadcasts and many radio frequency signals to be received and processed, often leading to - in case of a density of wearable or portable devices (and/or in case of a rate of radio frequency signals to be received and/or processed by each one of the wearable or portable devices considered) exceeding a certain density threshold - the wearable or portable devices broadcasting the radio frequency signals less often in such high density conditions, i.e. the above mentioned rate of broadcasts (under normal conditions) might be adaptively reduced in such high density conditions. However, each such broadcast event of a wearable or portable device involves the wearable or portable device transmitting or broadcasting its (at least temporarily) associated or assigned tag identifier (hereinafter also called tag identifier information).

Hence according to the present invention, in a contact situation of a specific wearable or portable device with a specific further wearable or portable device (or in a proximity event of these specific wearable or portable devices), the specific wearable or portable device is able, by means of the wearable or portable device's wireless communication interface and with respect to the specific further wearable or portable device, to determine repeatedly the distance, at different points in time, towards the specific further wearable or portable device and to receive (typically at a plurality of points in time) the specific further wearable or portable device's tag identifier. From these received broadcast pieces of information (that each wearable or portable device is able to receive from the other wearable or portable devices nearby, i.e. within the coverage area of the respective other wearable or portable device), related to different points in time, it is determined that the specific further wearable or portable device is positioned at most at, or below, the specific distance, from the specific wearable or portable device, and this for at least the specific time interval (or duration), wherein the proximity event is considered to be an encounter detection event if the specific distance corresponds to the risk distance threshold and the specific time interval corresponds to the risk time interval and/or wherein the proximity event is considered to be an alert event if the specific distance corresponds to the alert distance threshold and the specific time interval corresponds to the alert time interval.

In order to apply appropriate (threshold) values - adapted to the respective situation or type of situations of the wearable or portable devices involved - it is proposed according to the present invention that the specific wearable or portable device additionally detects or receives the group size information, being indicative of the relevant group size and/or of the relevant density of persons, and/or the environmental information, being indicative of a static and/or dynamic environmental parameter or property, and that the risk distance threshold as well as the risk time interval and/or the alert distance threshold as well as the alert time interval to be applied are dependent on the group size information and/or on the environmental information - thus leading to a more adaptive and more meaningful alerting behavior and/or risk assessment which is able to be tailored to the respective situation or type of situations of the wearable or portable devices involved.

According to the present invention, it is advantageously possible and preferred that the plurality of persons comprises at least a first and a second group of persons, wherein, during the first and/or second step, either the specific further wearable or portable device or still a further wearable or portable device is detected, by the specific wearable or portable device (itself belonging to the first group of persons), as belonging to the second group of persons, wherein especially a part of the tag identifiers relates to or defines the different groups of persons or is identical within the same group of persons.

By means of the specific wearable or portable device being able to determine that the specific further wearable or portable device (or still a further wearable or portable device) belongs to the second group of persons (instead to the same group of persons that also the specific wearable or portable device belongs to, i.e. the first group of persons), it is advantageously possible to take this information or piece of information into consideration regarding the alerting behavior and/or the risk assessment.

By means of a part of the tag identifiers relating to or defining the different groups of persons or being identical within the same group of persons, it is advantageously possible to quickly and directly determine the group identity (and, hence, the group) of the specific further wearable or portable device, especially without necessitating consulting a database; thereby, it is advantageously possible to be able to perform the determination of the group association (or which group a wearable or portable device belongs to) without having access to such a database or without having available such a database, i.e. especially this determination is able to be carried out in a de-centralized manner, and especially by the respective wearable or portable device itself, thus being able to be used for an adapted alerting behavior of the wearable or portable device based on locally available information.

According to the present invention, it is furthermore advantageously possible and preferred that the risk distance threshold and the risk time interval and/or the alert distance threshold and the alert time interval to be applied are also dependent - besides on the group size information and/or on the environmental information - on whether the specific further wearable or portable device or the still further wearable or portable device is detected as belonging to the first or the second groups of persons.

Thereby, it is advantageously possible to provide for an adaptation in the detection of alert situations and/or of relevant encounters dependent on whether a proximity event occurred within the same (first) group of persons, or whether also persons of another (second) group are or have been involved. Thereby, it is especially advantageously possible according to the present invention to be able to specifically account for non-local users (which are, e.g., given specific tags or wearable or portable devices with a system-wide known (or, alternatively, broadcasting an additional) parameter, so that other tags know that the bearer (i.e. the person wearing that wearable or portable device) is of the usual staff (of a company or organization (or whether the bearer is, e.g., a visitor).

According to the present invention, it is furthermore advantageously possible and preferred that the group size information corresponds to the number of different tag identifiers received or detected by the specific wearable or portable device while being in the contact situation with the specific further wearable or portable device, especially the group size information corresponds to this number of different tag identifiers within a predetermined group count time interval.

Thereby, it is advantageously possible to adapt the behavior of the detection of alert situations and/or of relevant encounters dependent on the group size information. Especially, the group size information corresponds to the number of different tag identifiers (received or detected by the specific wearable or portable device while being in the contact situation with the specific further wearable or portable device) plus one, as the specific wearable or portable device itself also belongs to the considered group of persons or wearable or portable devices. Especially, the group size information is detected ad hoc by the specific wearable or portable device (or tag).

According to the present invention, it is advantageously furthermore possible and preferred that the environmental information corresponds to at least one out of the following:
-- a static or quasi-static parameter or property information of or relating to the environment or setting of the specific wearable or portable device, as received or determined by the specific wearable or portable device, especially an information received from one fixedly installed tag device or from a plurality of fixedly installed tag devices, and indicating or referring to at least one possibility out of a plurality of different environments or settings or types of environments or settings,
-- a dynamic parameter or property information of or relating to the environment or setting of the specific wearable or portable device, as received or determined by the specific wearable or portable device, especially an information received from a sensor device, especially at least one out of the following: a CO₂-sensor, a temperature sensor, a nanoparticle sensor, an aerosol sensor, a humidity sensor.

Thereby, it is advantageously possible to take into account static and/or dynamic information regarding the environment of the specific wearable or portable device, for example the type of room or location the specific wearable or portable device is currently located. For example, a static parameter or property information might be broadcast by a fixedly installed tag device or from a plurality of fixedly installed tag devices, indicating, e.g., the (static) information "small room" by means of broadcasting a first value, and indicating, e.g., the (static) information "conference room" by means of broadcasting a second value, and indicating, e.g., the (static) information "ventilated hallway" by means of broadcasting a third value, and indicating, e.g., the (static) information "close collaboration unavoidable" by means of broadcasting a fourth value, etc.

According to the present invention, it is furthermore advantageously possible and preferred that the risk distance threshold and the risk time interval used correspond to a predetermined default risk distance threshold and a predetermined default risk time interval, respectively, being modified based on a first modification operation in dependency of the group size information and/or of the environmental information and/or of whether the specific further wearable or portable device or the still further wearable or portable device is detected as belonging to the first or second groups of persons, wherein the first modification operation corresponds to modifying the default risk distance threshold and/or the default risk time interval by means of, respectively, a discreet or continuous first modification value, the first modification value especially being added to the default risk distance threshold and/or to the default risk time interval or multiplied with the default risk distance threshold and/or with the default risk time interval.

According to a further preferred embodiment of the present invention, the alert distance threshold and the alert time interval actually applied correspond to a predetermined default alert distance threshold and a predetermined default alert time interval, respectively, being modified based on a second modification operation in dependency of the group size information and/or of the environmental information and/or of whether the specific further wearable or portable device or the still further wearable or portable device is detected as belonging to the first or second groups of persons, wherein the second modification operation corresponds to modifying the default alert distance threshold and/or the default alert time interval by means of, respectively, a discreet or continuous second modification value, the second modification value especially being added to the default alert distance threshold and/or to the default alert time interval or multiplied with the default alert distance threshold and/or with the default alert time interval.

By means of the first and/or second modification operation, it is advantageously possible according to the present invention to flexibly modify the respective default value such as to be able to apply the most appropriate values of the risk distance threshold and the risk time interval and/or of the alert distance threshold and the alert time interval.

Additionally, it is furthermore preferred that the predetermined default risk distance threshold is larger or equal to the predetermined default alert distance threshold and/or wherein the predetermined default risk time interval is larger or equal to the predetermined default alert time interval,
wherein especially the respective value or values of the predetermined default risk distance threshold and/or the predetermined default risk time interval and/or the predetermined default alert distance threshold and/or the predetermined default alert time interval is or are different between the specific wearable or portable device and the specific further wearable or portable device, based on the respective user's choice,
wherein such a value or such values are able to be modified by or involving the respective user of the wearable or portable device.

It is thereby advantageously possible to provide for a modification of the applicable threshold values based on personal preferences of the user of a respective wearable or portable device or tag, such personal preferences being informed e.g. by the respective person's health status or vulnerabilities of developing a severe outcome of an infection.

According to a further preferred embodiment of the present invention, the wearable or portable device is configured to store log data and to transmit the log data to an edge device associated to or assigned to the wearable or portable device.

Furthermore, the present invention relates to a system for enabling contact tracing and contact alerting among a plurality of persons by means of detecting or measuring the distance between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices, wherein, in a contact situation of a specific wearable or portable device with a specific further wearable or portable device, the specific wearable or portable device is able, by means of the specific wearable or portable device's wireless communication interface and with respect to the specific further wearable or portable device, to repeatedly determine the distance, at different points in time, towards the specific further wearable or portable device and to receive the specific further wearable or portable device's tag identifier, wherein, in order to detect alert situations and/or in order to detect relevant encounters or to assess the relevance of encounters of the specific wearable or portable device with the specific further wearable or portable device, the system is configured such that:
-- at least one proximity event involving the specific further wearable or portable device, and detected by the specific wearable or portable device, is determined by means of detecting the specific further wearable or portable device to be positioned at most at, or below, a specific distance, from the specific wearable or portable device, and this for at least a specific time interval, wherein the proximity event is considered to be an encounter detection event if the specific distance corresponds to a risk distance threshold and the specific time interval corresponds to a risk time interval and/or wherein the proximity event is considered to be an alert event if the specific distance corresponds to an alert distance threshold and the specific time interval corresponds to an alert time interval,
-- the specific wearable or portable device additionally, detects or receives
   -- a group size information, the group size information being indicative of the relevant group size and/or of the relevant density of persons, and/or
   -- an environmental information, the environmental information being indicative of a static and/or dynamic environmental parameter or property,
wherein the risk distance threshold and the risk time interval and/or the alert distance threshold and the alert time interval to be applied are dependent on the group size information and/or on the environmental information.

Furthermore, the present invention relates to tag device being part of an inventive system for enabling contact tracing and contact alerting among a plurality of persons by means of detecting or measuring the distance between two persons.

Additionally, the present invention relates to wearable or portable devices for enabling contact tracing and contact alerting among a plurality of persons by means of detecting or measuring the distance between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices,
wherein, in a contact situation of a specific wearable or portable device with a specific further wearable or portable device, the specific wearable or portable device is able, by means of the specific wearable or portable device's wireless communication interface and with respect to the specific further wearable or portable device, to repeatedly determine the distance, at different points in time, towards the specific further wearable or portable device and to receive the specific further wearable or portable device's tag identifier,
wherein, in order to detect alert situations and/or in order to detect relevant encounters or to assess the relevance of encounters of the specific wearable or portable device with the specific further wearable or portable device, the specific wearable or portable devices are configured such that:
   -- at least one proximity event involving the specific further wearable or portable device, and detected by the specific wearable or portable device, is determined by means of detecting the specific further wearable or portable device to be positioned at most at, or below, a specific distance, from the specific wearable or portable device, and this for at least a specific time interval, wherein the proximity event is considered to be an encounter detection event if the specific distance corresponds to a risk distance threshold and the specific time interval corresponds to a risk time interval and/or wherein the proximity event is considered to be an alert event if the specific distance corresponds to an alert distance threshold and the specific time interval corresponds to an alert time interval,
   -- the specific wearable or portable device additionally, detects or receives
      -- a group size information, the group size information being indicative of the relevant group size and/or of the relevant density of persons, and/or
      -- an environmental information, the environmental information being indicative of a static and/or dynamic environmental parameter or property,
wherein the risk distance threshold and the risk time interval and/or the alert distance threshold and the alert time interval to be applied are dependent on the group size information and/or on the environmental information.

Additionally, the present invention relates to a program comprising a computer readable program code which, when executed on a computer and/or on a wearable or portable device and/or on an edge device and/or on a computing device, or in part on a wearable or portable device and/or in part on an edge device and/or in part on a computing device, causes the computer and/or the wearable or portable device and/or the edge device and/or the computing device to perform the inventive method.

Still additionally, the present invention relates to a computer-readable medium comprising instructions which when executed on a computer and/or on a wearable or portable device and/or on an edge device and/or on a computing device, or in part on a wearable or portable device and/or in part on an edge device and/or in part on a computing device, causes the computer and/or the wearable or portable device and/or the edge device and/or the computing device to perform the inventive method.

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention. The reference figures quoted below refer to the attached drawings.

### BRIEF DESCRIPTION OF THE DRAWINGS

Figure 1 schematically illustrates a plurality of persons in a contact detection and/or contact tracing setting, wherein each of the plurality of persons is wearing or carrying a wearable or portable device (or tag) of a plurality of wearable or portable devices (or tags).
Figure 2 schematically illustrates a situation of a plurality of wearable or portable devices (or tags), one of these wearable or portable devices being connected to or transmitting data towards an edge device.
Figure 3 schematically illustrates two contact situations of a specific wearable or portable device with a specific further wearable or portable device, the specific wearable or portable device being able, by means of the specific wearable or portable device's wireless communication interface and with respect to the specific further wearable or portable device, to determine repeatedly the distance towards the specific further wearable or portable device and to receive the specific further wearable or portable device's tag identifier.

### DETAILED DESCRIPTION

The present invention will be described with respect to particular embodiments and with reference to certain drawings, but the invention is not limited thereto but only by the claims. The drawings described are only schematic and are nonlimiting. In the drawings, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated.

Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

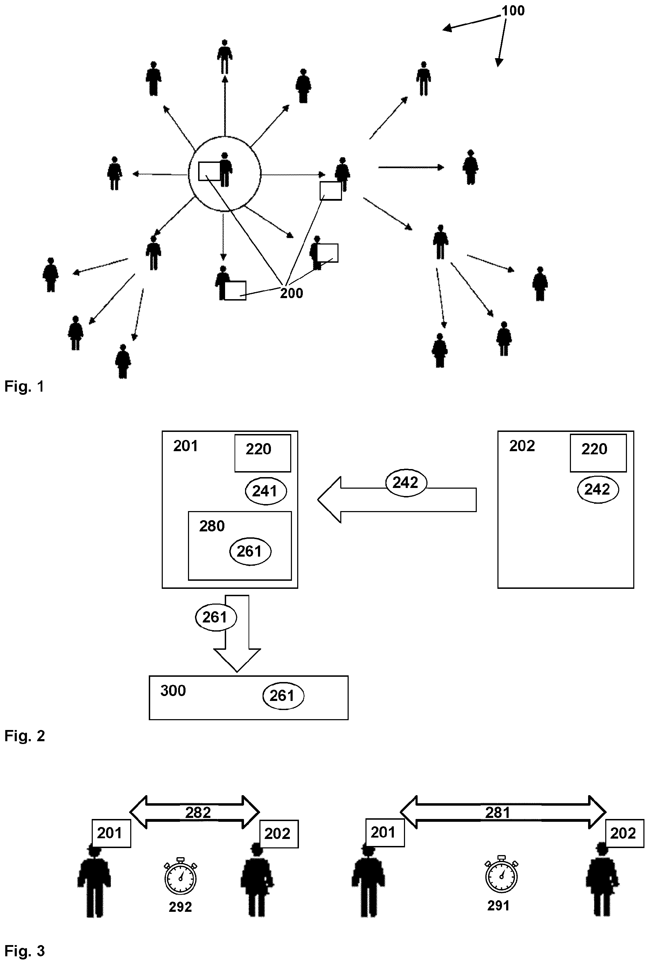

In Figure 1, a plurality of persons 100 in a contact detection and/or contact tracing setting are schematically represented, wherein each of the plurality of persons 100 is wearing or carrying a wearable or portable device (or tag) of a plurality of wearable or portable devices (or tags) 200, wherein for the sake of simplicity, Figure 1 only shows wearable or portable devices 200 for four persons 100 explicitly.

In Figure 2, a contact situation of a (specific) wearable or portable device 201 with a (specific) further wearable or portable device 202 is schematically shown. Each of the wearable or portable devices 201, 202 comprise, respectively, a wireless communication interface 220, and each of the wearable or portable devices 201, 202 comprise, respectively, a tag identifier, the wearable or portable device's tag identifier being indicated, in Figure 3, by means of reference sign 241, and the further wearable or portable device's tag identifier being indicated by means of reference sign 242. By means of the wearable or portable device's wireless communication interface 220, the wearable or portable device 201 is able, with respect to the further wearable or portable device 202, to determine repeatedly the or its distance (i.e. the distance of the wearable or portable device 201) towards the further wearable or portable device 202 and to receive the further wearable or portable device's tag identifier 242 (by means of receiving radio frequency signals broadcast by the further wearable or portable device 202 and comprising the further wearable or portable device's tag identifier 242), thereby generating and storing log data 261 of the contact situation of the wearable or portable device 201 with the further wearable or portable device 202. Of course, analogous contact situations will typically also arise with respect to still further wearable or portable devices (not individually indicated by means of references signs), i.e. the wearable or portable device 201 will typically also receive the broadcast signals of these still further wearable or portable devices, and, consequently, the log data 261 will typically also comprise information regarding the corresponding contact situations.

Figure 2 furthermore schematically shows the log data 261 of the wearable or portable device 201 being stored in a suitable memory device or module of the wearable or portable device 201 (i.e. the wearable or portable device 201 comprises such a suitable memory device or module 280, only represented in Figure 2 for the wearable or portable device 201) and being transferred to the edge device 300. According to one embodiment of the present invention, the log data 261 (of the wearable or portable device 201) are transferred to the edge device 300, typically once the wearable or portable device 201 is - after having been used by a person, e.g. during a work shift or during a working day - put back to or with the edge device 300, especially for purposes of charging (the batteries of the wearable or portable device 201).

Figure 3 schematically illustrates two contact situations of the wearable or portable device 201 with the further wearable or portable device 202, the wearable or portable device 201 being able, by means of the wearable or portable device's wireless communication interface 220 and with respect to the further wearable or portable device 202, to determine repeatedly the distance towards the further wearable or portable device 202 and to receive the further wearable or portable device's tag identifier 242.

According to the present invention, a method is proposed for enabling contact tracing and contact alerting among a plurality of persons 100 by means of detecting or measuring the distance between two persons. It is assumed that each of the plurality of persons 100 are wearing or carrying a wearable or portable device 201, 202, and the wearable or portable devices 201, 202 have or are associated or assigned to, respectively, a tag identifier 241, 242. By means of the wireless communication interface 220, respectively, of each one of the wearable or portable devices 201, 202, the tag identifiers 241, 242 are repeatedly broadcast by the wearable or portable devices 201, 202, respectively, such that - in a contact situation of the specific wearable or portable device 201 with the specific further wearable or portable device 202 - the wearable or portable device 201 is able, with respect to the further wearable or portable device 202, to repeatedly determine the distance, at different points in time, towards the further wearable or portable device 202 and to receive the further wearable or portable device's tag identifier 242. The same is, of course, true vice versa - however, in the following the invention is primarily described from the perspective of the wearable or portable device 201.

According to the present invention, in order to detect alert situations and/or in order to detect relevant encounters or to assess the relevance of encounters of the wearable or portable device 201 with the further wearable or portable device 202, the method comprises the steps of:
-- at least one proximity event involving the further wearable or portable device 202, and detected by the wearable or portable device 201, is determined by means of detecting the further wearable or portable device 202 to be positioned at most at, or below, a specific distance, from the wearable or portable device 201, and this for at least a specific time interval, wherein the proximity event is considered to be an encounter detection event if the specific distance corresponds to a risk distance threshold 281 and the specific time interval corresponds to a risk time interval 291 and/or wherein the proximity event is considered to be an alert event if the specific distance corresponds to an alert distance threshold 282 and the specific time interval corresponds to an alert time interval 292,
-- the wearable or portable device 201 additionally, detects or receives
   -- a group size information, the group size information being indicative of the relevant group size and/or of the relevant density of persons, and/or
   -- an environmental information, the environmental information being indicative of a static and/or dynamic environmental parameter or property,
wherein the risk distance threshold 281 and the risk time interval 291 and/or the alert distance threshold 282 and the alert time interval 292 to be applied are dependent on the group size information and/or on the environmental information.

This is schematically shown in Figure 3, where, on the left hand side, the wearable or portable device 201 is shown to be located at or below the alert distance threshold 282 from the further wearable or portable device 202, and this for (or during) at least the alert time interval 292. On the right hand side in Figure 3, the wearable or portable device 201 is shown to be located at or below the risk distance threshold 281 from the further wearable or portable device 202, and this for (or during) at least the risk time interval 291.

In conventionally known contact or encounter detection and/or alert systems, a static warning or alerting distance and a static warning or alerting duration are applied, i.e. by means of applying such static values, it is determined whether an alarm or alert, especially an acoustic alarm is activated, e.g. if two tags are closer than d_{warn} meters (e.g., say, 1,5 or 2 meters) for more than t_{warn} seconds (e.g., say, 5, 10 or 15 seconds), both tags involved start beeping. Likewise, risk calculation on the respective users' smartphones uses risk distance dᵣᵢₛₖ and risk duration tᵣᵢₛₖ, such that all encounters during one tag usage period are cumulatively counted or taken into consideration, i.e. in case that the encounters add up to tᵣᵢₛₖ minutes or more, the collection of encounters (between the two wearable or portable devices concerned) is deemed risky.

However in practice, there are many different situations and types of situations where the use of such static values or thresholds (for either warning or alerting and/or for assessing the (infection) risk) fall short of meaningfully reflecting the corresponding situation.

According to the present invention, it is, hence, advantageously possible to be able to take additional parameters and/or measurements into consideration regarding triggering an alert and/or for assessing the (infection) risk of a person wearing or carrying the wearable or portable device 201: Instead of simply using static values of a distance threshold value together with a time interval value such that:
-- a risk situation is defined to be detected if the distance to another wearable or portable device is detected to have fallen under such a static risk distance threshold value for a time interval corresponding at least to a static risk time interval value, and, correspondingly,
-- an alert situation is defined to be detected if the distance to another wearable or portable device is detected to have fallen under such a static alert distance threshold value for a time interval corresponding at least to a static alert time interval value,
it is proposed according to the present invention that the applicable threshold values (i.e. the risk distance threshold 281 together with the risk time interval 291 and/or the alert distance threshold 282 together with the alert time interval 292) are modified in a manner dependent on the group size information and/or the environmental information. Thereby, the group size information is indicative of the relevant group size and/or of the relevant density of persons, especially in the vicinity of the wearable or portable device 201, i.e. detectable by the wearable or portable device 201. The environmental information is indicative of a static and/or dynamic environmental parameter or property, likewise especially in the vicinity of the wearable or portable device 201.

Thereby, it is advantageously possible to apply threshold values that are more appropriate regarding different specific situations than just constant and average model parameters; hence, according to the present invention, it is advantageously possible to more realistically assess warning and alerting levels as well as risk assessment regarding encounters of individual users, taking into account different situations where the warning level and/or the infection risk is or are either higher or lower than supposed for average situations.

Preferably, it is possible, by the wearable or portable device 201, to determine whether the further wearable or portable device 202 belongs to the same (first) group (as the wearable or portable device 201) or whether the further wearable or portable device 202 belongs to a different group of persons, e.g. visitors that are present within a company's premises only sporadically. Such differentiation between different groups of persons is especially possible in an easy manner in case that a group is identified by means of a part of the respective tag identifiers which part is identical within the same group of persons (and different among different groups of persons).

Hence, it is especially preferred according to the present invention that the risk distance threshold 281 together with the risk time interval 291 and/or the alert distance threshold 282 together with the alert time interval 292 that are actually applied are dependent on the group size information and/or on the environmental information as well as on whether the specific further wearable or portable device 202 or the still further wearable or portable device (or the respective person) belongs to the same or a different group of persons.

According to the present invention, preferably the risk distance threshold 281 and the risk time interval 291 and/or the alert distance threshold 282 and the alert time interval 292 that are actually applied or used, correspond to predetermined default values (i.e. a predetermined default risk distance threshold and a predetermined default risk time interval in case of the risk distance threshold 281 and the risk time interval 291, and a predetermined default alert distance threshold and a predetermined default alert time interval in case of the alert distance threshold 282 and the alert time interval 292) that are, respectively, modified based on a modification operation in dependency of the group size information and/or of the environmental information and/or of whether the specific further wearable or portable device 202 or the still further wearable or portable device is detected as belonging to the first group (as the wearable or portable device 201) or, alternatively, to the second (i.e. different) group of persons. The term first modification operation is used for obtaining (or calculating) the risk distance threshold 281 and the risk time interval 291, whereas the term second modification operation is used for obtaining (or calculating) the alert distance threshold 282 and the alert time interval 292.

Hence, according to the present invention, environmental influences are taken into consideration for obtaining or calculating the risk distance threshold 281 / time interval 291 and/or the alert distance threshold 282 / time interval 292 actually applied in a specific situation of the wearable or portable device 201 and its environment.

Especially according to the present invention four categories of environmental influences that are fed differently into the system (or are taken into consideration):
-- First, group size, i.e. the number of people within scanning range of a tag (or wearable or portable device 201), plus one (i.e. the wearable or portable device 201 itself), i.e. corresponding to the group size information; group size is detected ad hoc by the tag (wearable or portable device 201) by simply counting the number of different tag IDs recognized within a given time interval (predetermined group count time interval), e.g. twenty seconds; the scanning frequency is usually between 0.1 Hz and 10 Hz.
-- Second, static environmental properties (i.e. a static or quasi-static parameter or property information of or relating to the environment or setting of the wearable or portable device 201, hence, an environmental information being static or quasi-static); these are represented, e.g., by fixed tags or tag devices (for example screwed to the wall) with fixed IDs (i.e. tag identifiers), so that a specific ID (i.e. broadcast tag identifier) stands for a specific property, independent from the specific location (i.e. no matter which customer or which facility); for instance, ID=1 might mean "small room", ID=2 might mean "conference room", ID=3 might mean "ventilated hallway", ID=4 might mean "close collaboration unavoidable", etc.
-- Third, dynamic environmental properties (i.e. a dynamic parameter or property information of or relating to the environment or setting of the wearable or portable device 201), hence, an environmental information being, potentially, dynamic); these are typically measured by sensors, e.g., CO₂ sensors, temperature sensors, nanoparticle sensors, aerosol sensors, humidity sensors, etc., which are typically connected to edge devices 300 and broadcast their measurements using a protocol which is readable by the tags, so that contact log entries on the tags or wearable or portable device 201, 202 (typically containing timestamp, the tag identifier of the other wearable or portable device, and a distance indication) are additionally annotated with sensor data.
-- Fourth, non-local users (i.e. the possibility to differentiate between different groups of persons such as, e.g., local users and non-local users); for example, non-local users are given specific tags (or wearable or portable devices) with a system-wide known ID range (or, alternatively, broadcasting an additional parameter), so that other tags (i.e. the specific wearable or portable device 201) know that the bearer (of the specific further wearable or portable device 202) is not one of the usual staff.
According to one embodiment of the present invention, for each of the four model parameters *d_{warn}, t_{warn}, dᵣᵢₛₖ,* and *tᵣᵢₛₖ,* a lower bound, a default value, and an upper bound is defined. Whenever a tag (or wearable or portable device) is picked up (by a person or bearer), the parameters are set to the default values. Then, each measurement or information provided by one of the four different pathways (that are available in near real time to each tag in the vicinity) has an effect on (a) the desirability of acoustic alarms or alerts (e.g., higher for large groups, hallways, and small rooms, lower for meeting rooms and certain work environments) and (b) the infection risk, e.g. for Covid-19, (e.g., higher for large groups and groups with non-local staff, lower for low aerosol density and outside events). In case (a), *d_{warn}* and *t_{warn}* are lowered or raised (individually or in tandem) but kept within the specified bounds. In case (b), *dᵣᵢₛₖ* and *tᵣᵢₛₖ* are lowered or raised (individually or in tandem) but kept within the specified bounds. The concrete formulas for adapting the model parameters are either binary ("if outdoors, set d = 3, otherwise, set d = 2"), or discreet, or continuous (linear or nonlinear) according to the respective configuration and/or adapting to customer needs and circumstances.

## Claims

1. Method for enabling contact tracing and contact alerting among a plurality of persons (100) by means of detecting or measuring the distance between two persons, wherein each of the plurality of persons (100) are wearing or carrying a wearable or portable device (201, 202), the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202),
wherein, in a contact situation of a specific wearable or portable device (201) with a specific further wearable or portable device (202), the wearable or portable device (201) is able, by means of the wearable or portable device's wireless communication interface (220) and with respect to the further wearable or portable device (202), to repeatedly determine the distance, at different points in time, towards the further wearable or portable device (202) and to receive the further wearable or portable device's tag identifier (242),
wherein, in order to detect alert situations and/or in order to detect relevant encounters or to assess the relevance of encounters of the wearable or portable device (201) with the further wearable or portable device (202), the method comprises the following steps:
-- in a first step, at least one proximity event involving the further wearable or portable device (202), and detected by the wearable or portable device (201), is determined by means of detecting the further wearable or portable device (202) to be positioned at most at, or below, a specific distance, from the wearable or portable device (201), and this for at least a specific time interval, wherein the proximity event is considered to be an encounter detection event if the specific distance corresponds to a risk distance threshold (281) and the specific time interval corresponds to a risk time interval (291) and/or wherein the proximity event is considered to be an alert event if the specific distance corresponds to an alert distance threshold (282) and the specific time interval corresponds to an alert time interval (292),
-- in a second step, prior to, during or after the first step, the wearable or portable device (201) additionally, detects or receives
-- a group size information, the group size information being indicative of the relevant group size and/or of the relevant density of persons, wherein the group size information corresponds to the number of different tag identifiers received or detected by the wearable or portable device (201) while being in the contact situation with the specific further wearable or portable device (202), and/or
-- an environmental information, the environmental information being indicative of a static and/or dynamic environmental parameter or property, wherein the environmental information corresponds to at least one out of the following:
-- a static or quasi-static parameter or property information as received by the wearable or portable device (201) from one fixedly installed tag device or from a plurality of fixedly installed tag devices, wherein the static or quasi-static parameter or property information relates to the environment and indicates at least one possibility out of a plurality of different environments or settings or types of environments or settings as received by the wearable or portable device (201),
-- a dynamic parameter or property information of or relating to the environment or setting of the wearable or portable device (201), as received by the wearable or portable device (201) from a sensor device,
wherein the risk distance threshold (281) and the risk time interval (291) and/or the alert distance threshold (282) and the alert time interval (292) to be applied are dependent on the group size information and/or on the environmental information.

2. Method according to claim 1, wherein the plurality of persons (100) comprises at least a first and a second group of persons, wherein, during the first and/or second step, either the specific further wearable or portable device (202) or still a further wearable or portable device is detected, by the specific wearable or portable device (201) belonging to the first group of persons, as belonging to the second group of persons, wherein especially a part of the tag identifiers relates to or defines the different groups of persons or is identical within the same group of persons.

3. Method according to one of the preceding claims, wherein the risk distance threshold (281) and the risk time interval (291) and/or the alert distance threshold (282) and the alert time interval (292) to be applied are also dependent - besides on the group size information and/or on the environmental information - on whether the specific further wearable or portable device (202) or the still further wearable or portable device is detected as belonging to the first or the second groups of persons.

4. Method according to one of the preceding claims, wherein the group size information corresponds to this number of different tag identifiers within a predetermined group count time interval.

5. Method according to one of the preceding claims, wherein the dynamic parameter or property information is at least one out of the following: a CO₂-sensor, a temperature sensor, a nanoparticle sensor, an aerosol sensor, a humidity sensor.

6. Method according to one of the preceding claims, wherein the risk distance threshold (281) and the risk time interval (291) used correspond to a predetermined default risk distance threshold and a predetermined default risk time interval, respectively, being modified based on a first modification operation in dependency of the group size information and/or of the environmental information and/or of whether the specific further wearable or portable device (202) or the still further wearable or portable device is detected as belonging to the first or second groups of persons,
wherein the first modification operation corresponds to modifying the default risk distance threshold and/or the default risk time interval by means of, respectively, a discreet or continuous first modification value, the first modification value especially being added to the default risk distance threshold and/or to the default risk time interval or multiplied with the default risk distance threshold and/or with the default risk time interval.

7. Method according to one of the preceding claims, wherein the alert distance threshold (282) and the alert time interval (292) actually applied correspond to a predetermined default alert distance threshold and a predetermined default alert time interval, respectively, being modified based on a second modification operation in dependency of the group size information and/or of the environmental information and/or of whether the specific further wearable or portable device (202) or the still further wearable or portable device is detected as belonging to the first or second groups of persons,
wherein the second modification operation corresponds to modifying the default alert distance threshold and/or the default alert time interval by means of, respectively, a discreet or continuous second modification value, the second modification value especially being added to the default alert distance threshold and/or to the default alert time interval or multiplied with the default alert distance threshold and/or with the default alert time interval.

8. Method according to one of the preceding claims, wherein the predetermined default risk distance threshold is larger or equal to the predetermined default alert distance threshold and/or wherein the predetermined default risk time interval is larger or equal to the predetermined default alert time interval,
wherein especially the respective value or values of the predetermined default risk distance threshold and/or the predetermined default risk time interval and/or the predetermined default alert distance threshold and/or the predetermined default alert time interval is or are different between the first and second wearable or portable device (201, 202), based on the respective user's choice, wherein such a value or such values are able to be modified by or involving the respective user of the wearable or portable device.

9. Method according to one of the preceding claims, wherein the wearable or portable device (201) is configured to store log data (261) and to transmit the log data (261) to an edge device (300) associated to or assigned to the wearable or portable device (201).

10. System for enabling contact tracing and contact alerting among a plurality of persons (100) by means of detecting or measuring the distance between two persons, wherein each of the plurality of persons (100) are wearing or carrying a wearable or portable device (201, 202), the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202),
wherein, in a contact situation of a specific wearable or portable device (201) with a specific further wearable or portable device (202), the wearable or portable device (201) is able, by means of the wearable or portable device's wireless communication interface (220) and with respect to the further wearable or portable device (202), to repeatedly determine the distance, at different points in time, towards the further wearable or portable device (202) and to receive the further wearable or portable device's tag identifier (242),
wherein, in order to detect alert situations and/or in order to detect relevant encounters or to assess the relevance of encounters of the wearable or portable device (201) with the further wearable or portable device (202), the system is configured such that:
-- at least one proximity event involving the further wearable or portable device (202), and detected by the wearable or portable device (201), is determined by means of detecting the further wearable or portable device (202) to be positioned at most at, or below, a specific distance, from the wearable or portable device (201), and this for at least a specific time interval, wherein the proximity event is considered to be an encounter detection event if the specific distance corresponds to a risk distance threshold (281) and the specific time interval corresponds to a risk time interval (291) and/or wherein the proximity event is considered to be an alert event if the specific distance corresponds to an alert distance threshold (282) and the specific time interval corresponds to an alert time interval (292),
-- the wearable or portable device (201) additionally, detects or receives
-- a group size information, the group size information being indicative of the relevant group size and/or of the relevant density of persons, wherein the group size information corresponds to the number of different tag identifiers received or detected by the wearable or portable device (201) while being in the contact situation with the specific further wearable or portable device (202), and/or
-- an environmental information, the environmental information being indicative of a static and/or dynamic environmental parameter or property, wherein the environmental information corresponds to at least one out of the following:
-- a static or quasi-static parameter or property information as received by the wearable or portable device (201) from one fixedly installed tag device or from a plurality of fixedly installed tag devices, wherein the static or quasi-static parameter or property information relates to the environment and indicates at least one possibility out of a plurality of different environments or settings or types of environments or settings as received by the wearable or portable device (201),
-- a dynamic parameter or property information of or relating to the environment or setting of the wearable or portable device (201), as received by the wearable or portable device (201) from a sensor device,
wherein the risk distance threshold (281) and the risk time interval (291) and/or the alert distance threshold (282) and the alert time interval (292) to be applied are dependent on the group size information and/or on the environmental information.

11. Wearable or portable devices (201, 202) for enabling contact tracing and contact alerting among a plurality of persons (100) by means of detecting or measuring the distance between two persons, wherein each of the plurality of persons (100) are wearing or carrying a wearable or portable device (201, 202), the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202),
wherein, in a contact situation of a specific wearable or portable device (201) with a specific further wearable or portable device (202), the wearable or portable device (201) is able, by means of the wearable or portable device's wireless communication interface (220) and with respect to the further wearable or portable device (202), to repeatedly determine the distance, at different points in time, towards the further wearable or portable device (202) and to receive the further wearable or portable device's tag identifier (242),
wherein, in order to detect alert situations and/or in order to detect relevant encounters or to assess the relevance of encounters of the wearable or portable device (201) with the further wearable or portable device (202), the wearable or portable devices (201, 202) are configured such that:
-- at least one proximity event involving the further wearable or portable device (202), and detected by the wearable or portable device (201), is determined by means of detecting the further wearable or portable device (202) to be positioned at most at, or below, a specific distance, from the wearable or portable device (201), and this for at least a specific time interval, wherein the proximity event is considered to be an encounter detection event if the specific distance corresponds to a risk distance threshold (281) and the specific time interval corresponds to a risk time interval (291) and/or wherein the proximity event is considered to be an alert event if the specific distance corresponds to an alert distance threshold (282) and the specific time interval corresponds to an alert time interval (292),
-- the wearable or portable device (201) additionally, detects or receives
-- a group size information, the group size information being indicative of the relevant group size and/or of the relevant density of persons, wherein the group size information corresponds to the number of different tag identifiers received or detected by the wearable or portable device (201) while being in the contact situation with the specific further wearable or portable device (202), and/or
-- an environmental information, the environmental information being indicative of a static and/or dynamic environmental parameter or property, wherein the environmental information corresponds to at least one out of the following:
-- a static or quasi-static parameter or property information as received by the wearable or portable device (201) from one fixedly installed tag device or from a plurality of fixedly installed tag devices, wherein the static or quasi-static parameter or property information relates to the environment and indicates at least one possibility out of a plurality of different environments or settings or types of environments or settings as received by the wearable or portable device (201),
-- a dynamic parameter or property information of or relating to the environment or setting of the wearable or portable device (201), as received by the wearable or portable device (201) from a sensor device,
wherein the risk distance threshold (281) and the risk time interval (291) and/or the alert distance threshold (282) and the alert time interval (292) to be applied are dependent on the group size information and/or on the environmental information.

12. Program comprising a computer readable program code, which, when executed on a computer and/or on a wearable or portable device (201) and/or on an edge device (300) and/or on a computing device (151), or in part on a wearable or portable device (201) and/or in part on an edge device (300) and/or in part on a computing device (151), causes the computer and/or the wearable or portable device (201) and/or the edge device (300) and/or the computing device (151) to perform a method according one of claims 1 to 9.

13. Computer-readable medium comprising instructions which when executed on a computer and/or on a wearable or portable device (201) and/or on an edge device (300) and/or on a computing device (151), or in part on a wearable or portable device (201) and/or in part on an edge device (300) and/or in part on a computing device (151), causes the computer and/or the wearable or portable device (201) and/or the edge device (300) and/or the computing device (151) to perform a method according one of claims 1 to 9.

## Patentansprüche

1. Verfahren zum Ermöglichen einer Kontaktverfolgung und Kontaktalarmierung unter mehreren Personen (100) mittels Detektieren oder Messen der Distanz zwischen zwei Personen, wobei jede der mehreren Personen (100) eine am Körper tragbare oder portable Vorrichtung (201, 202) trägt oder mit sich führt, wobei die am Körper tragbaren oder portablen Vorrichtungen (201, 202) eine Transponderkennung (241, 242) aufweisen oder mit einer Transponderkennung verknüpft bzw. einer Transponderkennung zugewiesen sind, wobei die Transponderkennungen (241, 242) wiederholt durch die am Körper tragbaren oder portablen Vorrichtungen (201, 202) unter Verwendung einer Drahtloskommunikationsschnittstelle der am Körper tragbaren oder portablen Vorrichtungen (201, 202) rundgesendet werden,
wobei in einer Kontaktsituation einer spezifischen am Körper tragbaren oder portablen Vorrichtung (201) mit einer spezifischen weiteren am Körper tragbaren oder portablen Vorrichtung (202) die am Körper tragbare oder portable Vorrichtung (201) in der Lage ist, mittels der Drahtloskommunikationsschnittstelle (220) der am Körper tragbaren oder portablen Vorrichtung und in Bezug auf die weitere am Körper tragbare oder portable Vorrichtung (202) wiederholt die Distanz, an verschiedenen Zeitpunkten, zu der weiteren am Körper tragbaren oder portablen Vorrichtung (202) zu bestimmen und die Transponderkennung (242) der weiteren am Körper tragbaren oder portablen Vorrichtung zu empfangen,
wobei das Verfahren, um Alarmsituationen zu detektieren und/oder um relevante Begegnungen der am Körper tragbaren oder portablen Vorrichtung (201) mit der weiteren am Körper tragbaren oder portablen Vorrichtung (202) zu detektieren oder um die Relevanz von Begegnungen der am Körper tragbaren oder portablen Vorrichtung (201) mit der weiteren am Körper tragbaren oder portablen Vorrichtung (202) zu beurteilen, die folgenden Schritte umfasst:
- in einem ersten Schritt wird mindestens ein Näherungsereignis, an dem die weitere am Körper tragbare oder portable Vorrichtung (202) beteiligt ist und das durch die am Körper tragbare oder portable Vorrichtung (201) detektiert wird, bestimmt, indem detektiert wird, dass die weitere am Körper tragbare oder portable Vorrichtung (202) maximal in einer, oder in weniger als einer, spezifischen Distanz von der am Körper tragbaren oder portablen Vorrichtung (201), und dies über mindestens ein spezifisches Zeitintervall, positioniert ist, wobei das Näherungsereignis als ein Begegnungsdetektionsereignis angesehen wird, falls die spezifische Distanz einer Risikodistanzschwelle (281) entspricht und das spezifische Zeitintervall einem Risikozeitintervall (291) entspricht, und/oder wobei das Näherungsereignis als ein Alarmereignis angesehen wird, falls die spezifische Distanz einer Alarmdistanzschwelle (282) entspricht und das spezifische Zeitintervall einem Alarmzeitintervall (292) entspricht,
- in einem zweiten Schritt, vor, während oder nach dem ersten Schritt, detektiert oder empfängt die am Körper tragbare oder portable Vorrichtung (201) zusätzlich
- eine Gruppengrößeninformation, wobei die Gruppengrößeninformation die relevante Gruppengröße und/oder die relevante Dichte von Personen angibt, wobei die Gruppengrößeninformation der Anzahl verschiedener Transponderkennungen entspricht, die durch die am Körper tragbare oder portable Vorrichtung (201) empfangen oder detektiert werden, während sie sich in der Kontaktsituation mit der spezifischen weiteren am Körper tragbaren oder portablen Vorrichtung (202) befindet, und/oder
- eine Umgebungsinformation, wobei die Umgebungsinformation einen statischen und/oder dynamischen Umgebungsparameter oder eine statische und/oder dynamische Umgebungseigenschaft angibt, wobei die Umgebungsinformation mindestens einem von Folgendem entspricht:
- einer statischen oder quasi-statischen Parameter- oder Eigenschaftsinformation, die durch die am Körper tragbare oder portable Vorrichtung (201) von einer fest installierten Transpondervorrichtung oder von mehreren fest installierten Transpondervorrichtungen empfangen werden, wobei sich die statische oder quasistatische Parameter- oder Eigenschaftsinformation auf die Umgebung bezieht und mindestens eine Möglichkeit aus mehreren verschiedenen Umgebungen oder Einstellungen oder Arten von Umgebungen oder Einstellungen angibt, die durch die am Körper tragbare oder portable Vorrichtung (201) empfangen werden,
- einer dynamischen Parameter- oder Eigenschaftsinformation der Umgebung oder Einstellung der am Körper tragbaren oder portablen Vorrichtung (201) oder in Bezug auf die Umgebung oder Einstellung der am Körper tragbaren oder portablen Vorrichtung (201), die durch die am Körper tragbare oder portable Vorrichtung (201) von einer Sensorvorrichtung empfangen wird,
wobei die Risikodistanzschwelle (281) und das Risikozeitintervall (291) und/oder die Alarmdistanzschwelle (282) und das Alarmzeitintervall (292), die anzuwenden sind, von der Gruppengrößeninformation und/oder von der Umgebungsinformation abhängig sind.

2. Verfahren nach Anspruch 1, wobei die mehreren Personen (100) mindestens eine erste und eine zweite Gruppe von Personen umfassen, wobei während des ersten und/oder des zweiten Schrittes entweder die spezifische weitere am Körper tragbare oder portable Vorrichtung (202) oder noch eine andere weitere tragbare oder portable Vorrichtung durch die spezifische am Körper tragbare oder portable Vorrichtung (201), die zu der der ersten Gruppe von Personen gehört, als zu der zweiten Gruppe von Personen gehörend detektiert wird, wobei insbesondere ein Teil der Transponderkennungen sich auf die verschiedenen Gruppen von Personen bezieht oder die verschiedenen Gruppen von Personen definiert oder innerhalb derselben Gruppe von Personen identisch ist.

3. Verfahren nach einem der vorangehenden Ansprüche, wobei die Risikodistanzschwelle (281) und das Risikozeitintervall (291) und/oder die Alarmdistanzschwelle (282) und das Alarmzeitintervall (292), die anzuwenden sind, des Weiteren nicht nur von der Gruppengrößeninformation und/oder der Umgebungsinformation abhängig sind, sondern auch davon, ob die spezifische weitere am Körper tragbare oder portable Vorrichtung (202) oder die andere weitere am Körper tragbare oder portable Vorrichtung als zu der ersten oder der zweiten Gruppe von Personen gehörend detektiert wird.

4. Verfahren nach einem der vorangehenden Ansprüche, wobei die Gruppengrößeninformation dieser Anzahl verschiedener Transponderkennungen innerhalb eines zuvor festgelegten Gruppenzählzeitintervalls entspricht.

5. Verfahren nach einem der vorangehenden Ansprüche, wobei die dynamische Parameter- oder Eigenschaftsinformation mindestens eines von Folgendem ist: ein CO₂-Sensor, ein Temperatursensor, ein Nanopartikelsensor, ein Aerosolsensor, ein Feuchtigkeitssensor.

6. Verfahren nach einem der vorangehenden Ansprüche, wobei die verwendete Risikodistanzschwelle (281) und das verwendete Risikozeitintervall (291) einer zuvor festgelegten Standard-Risikodistanzschwelle bzw. einem zuvor festgelegten Standard-Risikozeitintervall entsprechen, die auf der Grundlage einer ersten Modifikationsoperation in Abhängigkeit von der Gruppengrößeninformation und/oder der Umgebungsinformation und/oder davon, ob die spezifische weitere am Körper tragbare oder portable Vorrichtung (202) oder die andere weitere am Körper tragbare oder portable Vorrichtung als zu der ersten oder der zweiten Gruppe von Personen gehörend detektiert wird, modifiziert werden,
wobei die erste Modifikationsoperation dem Modifizieren der Standard-Risikodistanzschwelle und/oder des Standard-Risikozeitintervalls mittels eines diskreten bzw. kontinuierlichen ersten Modifikationswertes entspricht, wobei der erste Modifikationswert insbesondere zu der Standard-Risikodistanzschwelle und/oder dem Standard-Risikozeitintervall addiert oder mit der Standard-Risikodistanzschwelle und/oder dem Standard-Risikozeitintervall multipliziert wird.

7. Verfahren nach einem der vorangehenden Ansprüche, wobei die Alarmdistanzschwelle (282) und das Alarmzeitintervall (292), die tatsächlich angewendet werden, einer zuvor festgelegten Standard-Alarmdistanzschwelle bzw. einem zuvor festgelegten Standard-Alarmzeitintervall entsprechen, die auf der Grundlage einer zweiten Modifikationsoperation in Abhängigkeit von der Gruppengrößeninformation und/oder der Umgebungsinformation und/oder davon, ob die spezifische weitere am Körper tragbare oder portable Vorrichtung (202) oder die andere weitere am Körper tragbare oder portable Vorrichtung als zu der ersten oder der zweiten Gruppe von Personen gehörend detektiert wird, modifiziert werden,
wobei die zweite Modifikationsoperation dem Modifizieren der Standard-Alarmdistanzschwelle und/oder des Standard-Alarmzeitintervalls mittels eines diskreten bzw. kontinuierlichen zweiten Modifikationswertes entspricht, wobei der zweite Modifikationswert insbesondere zu der Standard-Alarmdistanzschwelle und/oder dem Standard-Alarmzeitintervall addiert oder mit der Standard-Alarmdistanzschwelle und/oder dem Standard-Alarmzeitintervall multipliziert wird.

8. Verfahren nach einem der vorangehenden Ansprüche, wobei die zuvor festgelegte Standard-Risikodistanzschwelle mindestens so groß wie die zuvor festgelegte Standard-Alarmdistanzschwelle ist und/oder wobei das zuvor festgelegte Standard-Risikozeitintervall mindestens so groß wie das zuvor festgelegte Standard-Alarmzeitintervall ist,
wobei insbesondere der jeweilige Wert oder die jeweiligen Werte der zuvor festgelegten Standard-Risikodistanzschwelle und/oder des zuvor festgelegten Standard-Risikozeitintervalls und/oder der zuvor festgelegten Standard-Alarmdistanzschwelle und/oder des zuvor festgelegten Standard-Alarmzeitintervalls zwischen der ersten und der zweiten am Körper tragbaren oder portablen Vorrichtung (201, 202) auf der Grundlage der jeweiligen Benutzerauswahl unterschiedlich sind, wobei ein solcher Wert oder solche Werte durch den jeweiligen Benutzer der am Körper tragbaren oder portablen Vorrichtung oder unter Einbeziehung des Benutzers der am Körper tragbaren oder portablen Vorrichtung modifiziert werden können.

9. Verfahren nach einem der vorangehenden Ansprüche, wobei die am Körper tragbare oder portable Vorrichtung (201) dafür eingerichtet ist, Log-Daten (261) zu speichern und die Log-Daten (261) an eine Rand-Vorrichtung (300) zu senden, die mit der am Körper tragbaren oder portablen Vorrichtung (201) verknüpft oder der am Körper tragbaren oder portablen Vorrichtung (201) zugeordnet ist.

10. System zum Ermöglichen einer Kontaktverfolgung und Kontaktalarmierung unter mehreren Personen (100) mittels Detektieren oder Messen der Distanz zwischen zwei Personen, wobei jede der mehreren Personen (100) eine am Körper tragbare oder portable Vorrichtung (201, 202) trägt oder mit sich führt, wobei die am Körper tragbaren oder portablen Vorrichtungen (201, 202) eine Transponderkennung (241, 242) aufweisen oder mit einer Transponderkennung verknüpft bzw. einer Transponderkennung zugewiesen sind, wobei die Transponderkennungen (241, 242) wiederholt durch die am Körper tragbaren oder portablen Vorrichtungen (201, 202) unter Verwendung einer Drahtloskommunikationsschnittstelle der am Körper tragbaren oder portablen Vorrichtungen (201, 202) rundgesendet werden,
wobei in einer Kontaktsituation einer spezifischen am Körper tragbaren oder portablen Vorrichtung (201) mit einer spezifischen weiteren am Körper tragbaren oder portablen Vorrichtung (202) die am Körper tragbare oder portable Vorrichtung (201) in der Lage ist, mittels der Drahtloskommunikationsschnittstelle (220) der am Körper tragbaren oder portablen Vorrichtung und in Bezug auf die weitere am Körper tragbare oder portable Vorrichtung (202) wiederholt die Distanz, an verschiedenen Zeitpunkten, zu der weiteren am Körper tragbaren oder portablen Vorrichtung (202) zu bestimmen und die Transponderkennung (242) der weiteren am Körper tragbaren oder portablen Vorrichtung zu empfangen,
wobei das System, um Alarmsituationen zu detektieren und/oder um relevante Begegnungen der am Körper tragbaren oder portablen Vorrichtung (201) mit der weiteren am Körper tragbaren oder portablen Vorrichtung (202) zu detektieren oder um die Relevanz von Begegnungen der am Körper tragbaren oder portablen Vorrichtung (201) mit der weiteren am Körper tragbaren oder portablen Vorrichtung (202) zu beurteilen, so eingerichtet ist, dass:
- mindestens ein Näherungsereignis, an dem die weitere am Körper tragbare oder portable Vorrichtung (202) beteiligt ist und das durch die am Körper tragbare oder portable Vorrichtung (201) detektiert wird, bestimmt wird, indem detektiert wird, dass die weitere am Körper tragbare oder portable Vorrichtung (202) maximal in einer, oder in weniger als einer, spezifischen Distanz von der am Körper tragbaren oder portablen Vorrichtung (201), und dies über mindestens ein spezifisches Zeitintervall, positioniert ist, wobei das Näherungsereignis als ein Begegnungsdetektionsereignis angesehen wird, falls die spezifische Distanz einer Risikodistanzschwelle (281) entspricht und das spezifische Zeitintervall einem Risikozeitintervall (291) entspricht, und/oder wobei das Näherungsereignis als ein Alarmereignis angesehen wird, falls die spezifische Distanz einer Alarmdistanzschwelle (282) entspricht und das spezifische Zeitintervall einem Alarmzeitintervall (292) entspricht,
- die am Körper tragbare oder portable Vorrichtung (201) zusätzlich detektiert oder empfängt:
- eine Gruppengrößeninformation, wobei die Gruppengrößeninformation die relevante Gruppengröße und/oder die relevante Dichte von Personen angibt, wobei die Gruppengrößeninformation der Anzahl verschiedener Transponderkennungen entspricht, die durch die am Körper tragbare oder portable Vorrichtung (201) empfangen oder detektiert werden, während sie sich in der Kontaktsituation mit der spezifischen weiteren am Körper tragbaren oder portablen Vorrichtung (202) befindet, und/oder
- eine Umgebungsinformation, wobei die Umgebungsinformation einen statischen und/oder dynamischen Umgebungsparameter oder eine statische und/oder dynamische Umgebungseigenschaft angibt, wobei die Umgebungsinformation mindestens einem von Folgendem entspricht:
- einer statischen oder quasi-statischen Parameter- oder Eigenschaftsinformation, die durch die am Körper tragbare oder portable Vorrichtung (201) von einer fest installierten Transpondervorrichtung oder von mehreren fest installierten Transpondervorrichtungen empfangen werden, wobei sich die statische oder quasistatische Parameter- oder Eigenschaftsinformation auf die Umgebung bezieht und mindestens eine Möglichkeit aus mehreren verschiedenen Umgebungen oder Einstellungen oder Arten von Umgebungen oder Einstellungen angibt, die durch die am Körper tragbare oder portable Vorrichtung (201) empfangen werden,
- einer dynamischen Parameter- oder Eigenschaftsinformation der Umgebung oder Einstellung der am Körper tragbaren oder portablen Vorrichtung (201) oder in Bezug auf die Umgebung oder Einstellung der am Körper tragbaren oder portablen Vorrichtung (201), die durch die am Körper tragbare oder portable Vorrichtung (201) von einer Sensorvorrichtung empfangen wird,
wobei die Risikodistanzschwelle (281) und das Risikozeitintervall (291) und/oder die Alarmdistanzschwelle (282) und das Alarmzeitintervall (292), die anzuwenden sind, von der Gruppengrößeninformation und/oder von der Umgebungsinformation abhängig sind.

11. Am Körper tragbare oder portable Vorrichtungen (201, 202) zum Ermöglichen einer Kontaktverfolgung und Kontaktalarmierung unter mehreren Personen (100) mittels Detektieren oder Messen der Distanz zwischen zwei Personen, wobei jede der mehreren Personen (100) eine am Körper tragbare oder portable Vorrichtung (201, 202) trägt oder mit sich führt, wobei die am Körper tragbaren oder portablen Vorrichtungen (201, 202) eine Transponderkennung (241, 242) aufweisen oder mit einer Transponderkennung verknüpft bzw. einer Transponderkennung zugewiesen sind, wobei die Transponderkennungen (241, 242) wiederholt durch die am Körper tragbaren oder portablen Vorrichtungen (201, 202) unter Verwendung einer Drahtloskommunikationsschnittstelle der am Körper tragbaren oder portablen Vorrichtungen (201, 202) rundgesendet werden,
wobei in einer Kontaktsituation einer spezifischen am Körper tragbaren oder portablen Vorrichtung (201) mit einer spezifischen weiteren am Körper tragbaren oder portablen Vorrichtung (202) die am Körper tragbare oder portable Vorrichtung (201) in der Lage ist, mittels der Drahtloskommunikationsschnittstelle (220) der am Körper tragbaren oder portablen Vorrichtung und in Bezug auf die weitere am Körper tragbare oder portable Vorrichtung (202) wiederholt die Distanz, an verschiedenen Zeitpunkten, zu der weiteren am Körper tragbaren oder portablen Vorrichtung (202) zu bestimmen und die Transponderkennung (242) der weiteren am Körper tragbaren oder portablen Vorrichtung zu empfangen,
wobei die am Körper tragbaren oder portablen Vorrichtungen (201, 202), um Alarmsituationen zu detektieren und/oder um relevante Begegnungen der am Körper tragbaren oder portablen Vorrichtung (201) mit der weiteren am Körper tragbaren oder portablen Vorrichtung (202) zu detektieren oder um die Relevanz von Begegnungen der am Körper tragbaren oder portablen Vorrichtung (201) mit der weiteren am Körper tragbaren oder portablen Vorrichtung (202) zu beurteilen, so eingerichtet sind, dass:
- mindestens ein Näherungsereignis, an dem die weitere am Körper tragbare oder portable Vorrichtung (202) beteiligt ist und das durch die am Körper tragbare oder portable Vorrichtung (201) detektiert wird, bestimmt wird, indem detektiert wird, dass die weitere am Körper tragbare oder portable Vorrichtung (202) maximal in einer, oder in weniger als einer, spezifischen Distanz von der am Körper tragbaren oder portablen Vorrichtung (201), und dies über mindestens ein spezifisches Zeitintervall, positioniert ist, wobei das Näherungsereignis als ein Begegnungsdetektionsereignis angesehen wird, falls die spezifische Distanz einer Risikodistanzschwelle (281) entspricht und das spezifische Zeitintervall einem Risikozeitintervall (291) entspricht, und/oder wobei das Näherungsereignis als ein Alarmereignis angesehen wird, falls die spezifische Distanz einer Alarmdistanzschwelle (282) entspricht und das spezifische Zeitintervall einem Alarmzeitintervall (292) entspricht,
- die am Körper tragbare oder portable Vorrichtung (201) zusätzlich detektiert oder empfängt:
- eine Gruppengrößeninformation, wobei die Gruppengrößeninformation die relevante Gruppengröße und/oder die relevante Dichte von Personen angibt, wobei die Gruppengrößeninformation der Anzahl verschiedener Transponderkennungen entspricht, die durch die am Körper tragbare oder portable Vorrichtung (201) empfangen oder detektiert werden, während sie sich in der Kontaktsituation mit der spezifischen weiteren am Körper tragbaren oder portablen Vorrichtung (202) befindet, und/oder
- eine Umgebungsinformation, wobei die Umgebungsinformation einen statischen und/oder dynamischen Umgebungsparameter oder eine statische und/oder dynamische Umgebungseigenschaft angibt, wobei die Umgebungsinformation mindestens einem von Folgendem entspricht:
- einer statischen oder quasi-statischen Parameter- oder Eigenschaftsinformation, die durch die am Körper tragbare oder portable Vorrichtung (201) von einer fest installierten Transpondervorrichtung oder von mehreren fest installierten Transpondervorrichtungen empfangen werden, wobei sich die statische oder quasistatische Parameter- oder Eigenschaftsinformation auf die Umgebung bezieht und mindestens eine Möglichkeit aus mehreren verschiedenen Umgebungen oder Einstellungen oder Arten von Umgebungen oder Einstellungen angibt, die durch die am Körper tragbare oder portable Vorrichtung (201) empfangen werden,
- einer dynamischen Parameter- oder Eigenschaftsinformation der Umgebung oder Einstellung der am Körper tragbaren oder portablen Vorrichtung (201) oder in Bezug auf die Umgebung oder Einstellung der am Körper tragbaren oder portablen Vorrichtung (201), die durch die am Körper tragbare oder portable Vorrichtung (201) von einer Sensorvorrichtung empfangen wird,
wobei die Risikodistanzschwelle (281) und das Risikozeitintervall (291) und/oder die Alarmdistanzschwelle (282) und das Alarmzeitintervall (292), die anzuwenden sind, von der Gruppengrößeninformation und/oder von der Umgebungsinformation abhängig sind.

12. Programm, umfassend einen computerlesbaren Programmcode, der, wenn er auf einem Computer und/oder in einer am Körper tragbaren oder portablen Vorrichtung (201) und/oder in einer Rand-Vorrichtung (300) und/oder in einer Computervorrichtung (151) oder teilweise in einer am Körper tragbaren oder portablen Vorrichtung (201) und/oder teilweise in einer Rand-Vorrichtung (300) und/oder teilweise in einer Computervorrichtung (151) ausgeführt wird, den Computer und/oder die am Körper tragbare oder portable Vorrichtung (201) und/oder die Rand-Vorrichtung (300) und/oder die Computervorrichtung (151) veranlasst, ein Verfahren nach einem der Ansprüche 1 bis 9 durchzuführen.

13. Computerlesbares Medium, umfassend Instruktionen, die, wenn sie auf einem Computer und/oder in einer am Körper tragbaren oder portablen Vorrichtung (201) und/oder in einer Rand-Vorrichtung (300) und/oder in einer Computervorrichtung (151) oder teilweise in einer am Körper tragbaren oder portablen Vorrichtung (201) und/oder teilweise in einer Rand-Vorrichtung (300) und/oder teilweise in einer Computervorrichtung (151) ausgeführt werden, den Computer und/oder die am Körper tragbare oder portable Vorrichtung (201) und/oder die Rand-Vorrichtung (300) und/oder die Computervorrichtung (151) veranlassen, ein Verfahren nach einem der Ansprüche 1 bis 9 durchzuführen.

## Revendications

1. Procédé destiné à permettre le traçage de contact et l'alerte de contact parmi une pluralité de personnes (100) au moyen de la détection ou de la mesure de la distance entre deux personnes, dans lequel chacune de la pluralité de personnes (100) porte ou transporte un dispositif portatif ou portable (201, 202), les dispositifs portatifs ou portables (201, 202) ayant ou étant associés ou attribués à, respectivement, un identificateur d'étiquette (241, 242), les identificateurs d'étiquettes (241, 242) étant diffusés de manière répétée par les dispositifs portatifs ou portables (201, 202), respectivement, à l'aide d'une interface de communication sans fil des dispositifs portatifs ou portables (201, 202),
dans lequel, dans une situation de contact d'un dispositif portatif ou portable spécifique (201) avec un autre dispositif portatif ou portable spécifique (202), le dispositif portatif ou portable (201) est capable, au moyen de l'interface de communication sans fil du dispositif portatif ou portable (220) et par rapport à l'autre dispositif portatif ou portable (202), de déterminer de manière répétée la distance, à différents moments dans le temps, vers l'autre dispositif portatif ou portable (202) et pour recevoir l'identificateur d'étiquette de l'autre dispositif portatif ou portable (242),
dans lequel, afin de détecter des situations d'alerte et/ou afin de détecter des rencontres pertinentes ou d'évaluer la pertinence de rencontres du dispositif portatif ou portable (201) avec l'autre dispositif portatif ou portable (202), le procédé comprend les étapes suivantes :
- dans une première étape, au moins un événement de proximité impliquant l'autre dispositif portatif ou portable (202), et détecté par le dispositif portatif ou portable (201), est déterminé au moyen de la détection du fait que l'autre dispositif portatif ou portable (202) est positionné au maximum à, ou en dessous, d'une distance spécifique, du dispositif portatif ou portable (201), et ce pendant au moins un intervalle de temps spécifique, dans lequel l'événement de proximité est considéré comme un événement de détection de rencontre si la distance spécifique correspond à un seuil de distance de risque (281) et l'intervalle de temps spécifique correspond à un intervalle de temps de risque (291) et/ou dans lequel l'événement de proximité est considéré comme un événement d'alerte si la distance spécifique correspond à un seuil de distance d'alerte (282) et l'intervalle de temps spécifique correspond à un intervalle de temps d'alerte (292),
- dans une seconde étape, avant, pendant ou après la première étape, le dispositif portatif ou portable (201) détecte ou reçoit en outre
- une information de taille de groupe, l'information de taille de groupe étant indicative de la taille de groupe pertinente et/ou de la densité de personnes pertinente, dans lequel l'information de taille de groupe correspond au nombre d'identificateurs d'étiquettes différents reçus ou détectés par le dispositif portatif ou portable (201) tout en étant dans la situation de contact avec l'autre dispositif portatif ou portable spécifique (202), et/ou
- une information environnementale, l'information environnementale étant indicative d'un paramètre ou d'une propriété environnemental(e) statique et/ou dynamique, dans lequel l'information environnementale correspond à au moins l'un parmi :
- une information de paramètre ou de propriété statique ou quasi-statique telle que reçue par le dispositif portatif ou portable (201) à partir d'un dispositif d'étiquette installé de manière fixe ou d'une pluralité de dispositifs d'étiquettes installés de manière fixe, dans lequel l'information de paramètre ou de propriété statique ou quasi-statique se rapporte à l'environnement et indique au moins une possibilité parmi une pluralité d'environnements ou de milieux ou de types d'environnements ou de milieux différents telle que reçue par le dispositif portatif ou portable (201),
- une information de paramètre ou de propriété dynamique de l'environnement ou du milieu du dispositif portatif ou portable (201), ou se rapportant à celui-ci, telle que reçue par le dispositif portatif ou portable (201) à partir d'un dispositif capteur, dans lequel le seuil de distance de risque (281) et l'intervalle de temps de risque (291) et/ou le seuil de distance d'alerte (282) et l'intervalle de temps d'alerte (292) à appliquer dépendent de l'information de taille de groupe et/ou de l'information environnementale.

2. Procédé selon la revendication 1, dans lequel la pluralité de personnes (100) comprend au moins un premier et un second groupe de personnes, dans lequel, pendant la première et/ou la seconde étape, soit l'autre dispositif portatif ou portable spécifique (202), soit encore un autre dispositif portatif ou portable est détecté, par le dispositif portatif ou portable spécifique (201) appartenant au premier groupe de personnes, comme appartenant au second groupe de personnes, dans lequel en particulier une partie des identificateurs d'étiquettes se rapporte à ou définit les différents groupes de personnes ou est identique au sein d'un même groupe de personnes.

3. Procédé selon l'une des revendications précédentes, dans lequel le seuil de distance de risque (281) et l'intervalle de temps de risque (291) et/ou le seuil de distance d'alerte (282) et l'intervalle de temps d'alerte (292) à appliquer dépendent également - en plus de l'information de taille de groupe et/ou de l'information environnementale - du fait de savoir si l'autre dispositif portatif ou portable spécifique (202) ou l'encore un autre dispositif portatif ou portable est détecté comme appartenant au premier ou au second groupe de personnes.

4. Procédé selon l'une des revendications précédentes, dans lequel l'information de taille de groupe correspond à ce nombre d'identificateurs d'étiquettes différents dans un intervalle de temps de comptage de groupe prédéterminé.

5. Procédé selon l'une des revendications précédentes, dans lequel l'information de propriété ou de paramètre dynamique est au moins l'un parmi ce qui suit : un capteur de CO₂, un capteur de température, un capteur de nanoparticules, un capteur d'aérosol, un capteur d'humidité.

6. Procédé selon l'une des revendications précédentes, dans lequel le seuil de distance de risque (281) et l'intervalle de temps de risque (291) utilisés correspondent respectivement à un seuil de distance de risque par défaut prédéterminé et à un intervalle de temps de risque par défaut prédéterminé, qui sont modifiés sur la base d'une première opération de modification en fonction de l'information de taille de groupe et/ou de l'information environnementale et/ou du fait de savoir si l'autre dispositif portatif ou portable spécifique (202) ou l'encore un autre dispositif portatif ou portable est détecté comme appartenant au premier ou au second groupe de personnes,
dans lequel la première opération de modification correspond à la modification du seuil de distance de risque par défaut et/ou de l'intervalle de temps de risque par défaut au moyen, respectivement, d'une première valeur de modification discrète ou continue, la première valeur de modification étant notamment ajoutée au seuil de distance de risque par défaut et/ou à l'intervalle de temps de risque par défaut ou multipliée par le seuil de distance de risque par défaut et/ou par l'intervalle de temps de risque par défaut.

7. Procédé selon l'une des revendications précédentes, dans lequel le seuil de distance d'alerte (282) et l'intervalle de temps d'alerte (292) effectivement appliqués correspondent respectivement à un seuil de distance d'alerte par défaut prédéterminé et à un intervalle de temps d'alerte par défaut prédéterminé, qui sont modifiés sur la base d'une seconde opération de modification en fonction de l'information de taille de groupe et/ou de l'information environnementale et/ou du fait de savoir si l'autre dispositif portatif ou portable spécifique (202) ou l'encore un autre dispositif portatif ou portable est détecté comme appartenant au premier ou au second groupe de personnes,
dans lequel la seconde opération de modification correspond à la modification du seuil de distance d'alerte par défaut et/ou à l'intervalle de temps d'alerte par défaut au moyen, respectivement, d'une seconde valeur de modification discrète ou continue, la seconde valeur de modification étant notamment ajoutée au seuil de distance d'alerte par défaut et/ou à l'intervalle de temps d'alerte par défaut ou multipliée par le seuil de distance d'alerte par défaut et/ou par l'intervalle de temps d'alerte par défaut.

8. Procédé selon l'une des revendications précédentes, dans lequel le seuil de distance de risque par défaut prédéterminé est supérieur ou égal au seuil de distance d'alerte par défaut prédéterminé et/ou dans lequel l'intervalle de temps de risque par défaut prédéterminé est supérieur ou égal à l'intervalle de temps d'alerte par défaut prédéterminé,
dans lequel notamment la ou les valeurs respectives du seuil de distance de risque par défaut prédéterminé et/ou de l'intervalle de temps de risque par défaut prédéterminé et/ou du seuil de distance d'alerte par défaut prédéterminé et/ou de l'intervalle de temps d'alerte par défaut prédéterminé sont différentes entre le premier et le second dispositif portatif ou portable (201, 202), sur la base du choix de l'utilisateur respectif, dans lequel une telle valeur ou de telles valeurs peuvent être modifiées par ou impliquant l'utilisateur respectif du dispositif portatif ou portable.

9. Procédé selon l'une des revendications précédentes, dans lequel le dispositif portatif ou portable (201) est configuré pour stocker des données de journal (261) et pour transmettre les données de journal (261) à un dispositif périphérique (300) associé ou attribué au dispositif portatif ou portable (201).

10. Système destiné à permettre traçage de contact et l'alerte de contact parmi une pluralité de personnes (100) au moyen de la détection ou de la mesure de la distance entre deux personnes, dans lequel chacune de la pluralité de personnes (100) porte ou transporte un dispositif portatif ou portable (201, 202), les dispositifs portatifs ou portables (201, 202) ayant ou étant associés ou attribués à, respectivement, un identificateur d'étiquette (241, 242), les identificateurs d'étiquettes (241, 242) étant diffusés de manière répétée par les dispositifs portatifs ou portables (201, 202), respectivement, à l'aide d'une interface de communication sans fil des dispositifs portatifs ou portables (201, 202),
dans lequel, dans une situation de contact d'un dispositif portatif ou portable spécifique (201) avec un autre dispositif portatif ou portable spécifique (202), le dispositif portatif ou portable (201) est capable, au moyen de l'interface de communication sans fil du dispositif portatif ou portable (220) et par rapport à l'autre dispositif portatif ou portable (202), de déterminer de manière répétée la distance, à différents moments dans le temps, vers l'autre dispositif portatif ou portable (202) et pour recevoir l'identificateur d'étiquette de l'autre dispositif portatif ou portable (242),
dans lequel, afin de détecter des situations d'alerte et/ou afin de détecter des rencontres pertinentes ou d'évaluer la pertinence de rencontres du dispositif portatif ou portable (201) avec l'autre dispositif portatif ou portable (202), le système est configuré de telle sorte que :
- au moins un événement de proximité impliquant l'autre dispositif portatif ou portable (202), et détecté par le dispositif portatif ou portable (201), est déterminé au moyen de la détection du fait que l'autre dispositif portatif ou portable (202) est positionné au maximum à, ou en dessous, d'une distance spécifique, du dispositif portatif ou portable (201), et ce pendant au moins un intervalle de temps spécifique, dans lequel l'événement de proximité est considéré comme un événement de détection de rencontre si la distance spécifique correspond à un seuil de distance de risque (281) et l'intervalle de temps spécifique correspond à un intervalle de temps de risque (291) et/ou dans lequel l'événement de proximité est considéré comme un événement d'alerte si la distance spécifique correspond à un seuil de distance d'alerte (282) et l'intervalle de temps spécifique correspond à un intervalle de temps d'alerte (292),
- le dispositif portatif ou portable (201) détecte ou reçoit en outre
- une information de taille de groupe, l'information de taille de groupe étant indicative de la taille de groupe pertinente et/ou de la densité de personnes pertinente, dans lequel l'information de taille de groupe correspond au nombre d'identificateurs d'étiquettes différents reçus ou détectés par le dispositif portatif ou portable (201) tout en étant dans la situation de contact avec l'autre dispositif portatif ou portable spécifique (202), et/ou
- une information environnementale, l'information environnementale étant indicative d'un paramètre ou d'une propriété environnemental(e) statique et/ou dynamique, dans lequel l'information environnementale correspond à au moins l'un parmi :
- une information de paramètre ou de propriété statique ou quasi-statique telle que reçue par le dispositif portatif ou portable (201) à partir d'un dispositif d'étiquette installé de manière fixe ou d'une pluralité de dispositifs d'étiquettes installés de manière fixe, dans lequel l'information de paramètre ou de propriété statique ou quasi-statique se rapporte à l'environnement et indique au moins une possibilité parmi une pluralité d'environnements ou de milieux ou de types d'environnements ou de milieux différents telle que reçue par le dispositif portatif ou portable (201),
- une information de paramètre ou de propriété dynamique de l'environnement ou du milieu du dispositif portatif ou portable (201), ou se rapportant à celui-ci, telle que reçue par le dispositif portatif ou portable (201) à partir d'un dispositif capteur, dans lequel le seuil de distance de risque (281) et l'intervalle de temps de risque (291) et/ou le seuil de distance d'alerte (282) et l'intervalle de temps d'alerte (292) à appliquer dépendent de l'information de taille de groupe et/ou de l'information environnementale.

11. Dispositifs portatifs ou portables (201, 202) destinés à permettre traçage de contact et l'alerte de contact parmi une pluralité de personnes (100) au moyen de la détection ou de la mesure de la distance entre deux personnes, dans lequel chacune de la pluralité de personnes (100) porte ou transporte un dispositif portatif ou portable (201, 202), les dispositifs portatifs ou portables (201, 202) ayant ou étant associés ou attribués à, respectivement, un identificateur d'étiquette (241, 242), les identificateurs d'étiquettes (241, 242) étant diffusés de manière répétée par les dispositifs portatifs ou portables (201, 202), respectivement, à l'aide d'une interface de communication sans fil des dispositifs portatifs ou portables (201, 202), dans lequel, dans une situation de contact d'un dispositif portatif ou portable spécifique (201) avec un autre dispositif portatif ou portable spécifique (202), le dispositif portatif ou portable (201) est capable, au moyen de l'interface de communication sans fil du dispositif portatif ou portable (220) et par rapport à l'autre dispositif portatif ou portable (202), de déterminer de manière répétée la distance, à différents moments dans le temps, vers l'autre dispositif portatif ou portable (202) et pour recevoir l'identificateur d'étiquette de l'autre dispositif portatif ou portable (242),
dans lequel, afin de détecter des situations d'alerte et/ou afin de détecter des rencontres pertinentes ou d'évaluer la pertinence de rencontres du dispositif portatif ou portable (201) avec l'autre dispositif portatif ou portable (202), les dispositifs portatifs ou portables (201, 202) sont configurés de telle sorte que :
- au moins un événement de proximité impliquant l'autre dispositif portatif ou portable (202), et détecté par le dispositif portatif ou portable (201), est déterminé au moyen de la détection du fait que l'autre dispositif portatif ou portable (202) est positionné au maximum à, ou en dessous, d'une distance spécifique, du dispositif portatif ou portable (201), et ce pendant au moins un intervalle de temps spécifique, dans lequel l'événement de proximité est considéré comme un événement de détection de rencontre si la distance spécifique correspond à un seuil de distance de risque (281) et l'intervalle de temps spécifique correspond à un intervalle de temps de risque (291) et/ou dans lequel l'événement de proximité est considéré comme un événement d'alerte si la distance spécifique correspond à un seuil de distance d'alerte (282) et l'intervalle de temps spécifique correspond à un intervalle de temps d'alerte (292),
- le dispositif portatif ou portable (201) détecte ou reçoit en outre
- une information de taille de groupe, l'information de taille de groupe étant indicative de la taille de groupe pertinente et/ou de la densité de personnes pertinente, dans lequel l'information de taille de groupe correspond au nombre d'identificateurs d'étiquettes différents reçus ou détectés par le dispositif portatif ou portable (201) tout en étant dans la situation de contact avec l'autre dispositif portatif ou portable spécifique (202), et/ou
- une information environnementale, l'information environnementale étant indicative d'un paramètre ou d'une propriété environnemental(e) statique et/ou dynamique, dans lequel l'information environnementale correspond à au moins l'un parmi :
- une information de paramètre ou de propriété statique ou quasi-statique telle que reçue par le dispositif portatif ou portable (201) à partir d'un dispositif d'étiquette installé de manière fixe ou d'une pluralité de dispositifs d'étiquettes installés de manière fixe, dans lequel l'information de paramètre ou de propriété statique ou quasi-statique se rapporte à l'environnement et indique au moins une possibilité parmi une pluralité d'environnements ou de milieux ou de types d'environnements ou de milieux différents telle que reçue par le dispositif portatif ou portable (201),
- une information de paramètre ou de propriété dynamique de l'environnement ou du milieu du dispositif portatif ou portable (201), ou se rapportant à celui-ci, telle que reçue par le dispositif portatif ou portable (201) à partir d'un dispositif capteur, dans lequel le seuil de distance de risque (281) et l'intervalle de temps de risque (291) et/ou le seuil de distance d'alerte (282) et l'intervalle de temps d'alerte (292) à appliquer dépendent de l'information de taille de groupe et/ou de l'information environnementale.

12. Programme comprenant un code de programme lisible par ordinateur qui, lorsqu'il est exécuté sur un ordinateur et/ou sur un dispositif portatif ou portable (201) et/ou sur un dispositif périphérique (300) et/ou sur un dispositif informatique (151), ou en partie sur un dispositif portatif ou portable (201) et/ou en partie sur un dispositif périphérique (300) et/ou en partie sur un dispositif informatique (151), amène l'ordinateur et/ou le dispositif portatif ou portable (201) et/ou le dispositif périphérique (300) et/ou le dispositif informatique (151) à mettre en œuvre un procédé selon l'une des revendications 1 à 9.

13. Support lisible par ordinateur comprenant des instructions qui, lorsqu'elles sont exécutées sur un ordinateur et/ou sur un dispositif portatif ou portable (201) et/ou sur un dispositif périphérique (300) et/ou sur un dispositif informatique (151), ou en partie sur un dispositif portatif ou portable (201) et/ou en partie sur un dispositif périphérique (300) et/ou en partie sur un dispositif informatique (151), amènent l'ordinateur et/ou le dispositif portatif ou portable (201) et/ou le dispositif périphérique (300) et/ou le dispositif informatique (151) à exécuter un procédé selon l'une des revendications 1 à 9.
